# EUROPEAN PATENT SPECIFICATION

(11) **EP 3 689 409 B1**
(45) Date of publication and mention of the grant of the patent: **18.10.2023**
(21) Application number: 18904970.3
(22) Date of filing: 21.09.2018
(51) Int. Cl.: A61M 25/02, A61M 25/082, A61M 25/09, A61M 39/08

(54) **MEDICAL ASSISTANCE DEVICE**
MEDIZINISCHE UNTERSTÜTZUNGSVORRICHTUNG
DISPOSITIF D'ASSISTANCE MÉDICALE

(30) Priority: 07.02.2018 JP 2018020554
(43) Date of publication of application: 05.08.2020
(73) Proprietor: Terumo Kabushiki Kaisha, Tokyo 151-0072 (JP)
(72) Inventor: KUSU Kohtaroh, Fujinomiya-shi Shizuoka 418-0015 (JP); YASUNAGA Mitsuteru, Fujinomiya-shi Shizuoka 418-0015 (JP); NOMURA Atsushi, Tokyo 163-1450 (JP); SEKINE Yusuke, Ashigarakami-gun Kanagawa 259-0151 (JP)
(74) Representative: Casalonga
(86) International application number: PCT/JP2018/034994
(87) International publication number: WO 2019/155677

(56) References cited:
- EP-A1- 2 386 327
- WO-A1-2008/074039
- WO-A1-2008/074039
- JP-A- S63 162 495
- JP-A- 2005 514 095
- JP-A- 2016 510 606
- JP-U- H0 675 507
- US-A1- 2010 307 496
- US-A1- 2011 011 401
- US-A1- 2011 028 941

## Description

### Technical Field

The present invention relates to a medical assistance device which is connected to a medical device used in a procedure in a biological lumen.

### Background Art

In recent years, for example, in a procedure such as a treatment or a diagnosis in a biological lumen such as a blood vessel, a bile duct, a trachea, an esophagus, or a urethra, a catheter treatment which is less invasive than a surgical operation has been recommended. For example, according to a site (puncture site) where a catheter is inserted into a human body, the catheter treatment is classified into a Trans-Radial Intervention (TRI), a Trans-Femoral Intervention (TFI), or the like.

In general, a blood vessel diameter of a radial artery is smaller than a blood vessel diameter of a femoral artery. Moreover, a speed of a blood flow in a blood vessel and a blood pressure in a blood vessel in the radial artery are different from those of the femoral artery. Therefore, compared with the Trans-Femoral Intervention, the Trans-Radial Intervention has less bleeding at the puncture site. Accordingly, in the Trans-Radial Intervention, hemostasis at the puncture site is easy, and a rest restraint time can be shortened. Therefore, according to a medical condition, a period from hospitalization to discharge can be shortened, or day surgery can be realized. In this way, the Trans-Radial Intervention can reduce a physical burden or an economic burden on a patient. As a result, superiority of the Trans-Radial Intervention is recognized as compared to the Trans-Femoral Intervention.

In a Percutaneous Coronary Intervention (PCI: coronary angioplasty), a thin tube called a "catheter" is inserted from a femoral artery of a lower limb, a radial artery of an upper limb, or a brachial artery and is delivered to a stenosed site of a coronary artery through an aorta. In this way, the procedure in the blood vessel of the coronary artery is performed. In this case, for example, a length of the catheter for delivering a procedure device to a procedure site such as a lesion area is 100 cm to 110 cm. For example, a length of the procedure device is 120 cm to 135 cm. A length of a guide wire which is used together with the catheter and the procedure device can be as long as 180 cm to 280 cm. Note that the length of each medical device is an example and may be changed according to a height and a body type of a patient.

Meanwhile, in the related art, in an Endovascular Treatment (EVT), the Trans-Femoral Intervention is adopted in a procedure in a biological lumen (peripheral blood vessel) of a lower limb. In the case of the Trans-Femoral Intervention, there are an ipsilateral approach which approaches from the same foot as a foot where a procedure site exists and a contralateral approach which approaches from the other foot opposite to the foot where the procedure site exists. For example, in the ipsilateral approach, a length of a catheter which delivers a procedure device to the procedure site is 45 cm. For example, a length of the procedure device is 80 cm. A length of a guide wire which is used together with the catheter and the procedure device can be as long as 180 cm. Similarly, in the contralateral approach, for example, a length of a catheter which delivers a procedure device to the procedure site is 90 cm. For example, a length of the procedure device is 135 cm. A length of a guide wire which is used together with the catheter and the procedure device can be as long as 260 cm.

However, even in the procedure in the biological lumen of the lower limb, when the Trans-Radial Intervention is to be adopted in consideration of a medical economy and an improvement in a Quality Of Life (QOL) of a patient, for example, it is assumed that the length of the catheter delivering the procedure device to the procedure site is 150 cm. For example, it is assumed that the length of the procedure device is 200 cm. It is assumed that the length of the guide wire which is used together with the catheter and the procedure device can be as long as 380 cm. In this way, when the Trans-Radial Intervention is to be adopted in the procedure in the biological lumen of the lower limb, the length of each medical device may not only be longer than the height of the patient or the height of a surgeon, but may also be longer than a catheter table of which an entire length in a longitudinal direction is about 330 cm. Moreover, a distance from the puncture site to the procedure site is changed depending on an age, a height, or a body type of the patient. Accordingly, the length of the existing medical device may be too long or too short for the procedure in the target biological lumen. That is, the length of the existing medical device may not match the procedure in the target biological lumen.

PTL 1 discloses a device which easily treats a damaged blood vessel of a patient. The device described in PTL 1 includes an extension wire. The extension wire has a coupling member for coupling to a corresponding coupling member of an access wire balloon catheter device used to occlude a blood flow in the damaged blood vessel, at one end of the extension wire. PTL 2 discloses a device for controlling the operating length of a catheter.

### Citation List

### Patent Literature

PTL 1: JP-T-2015-531283
PTL 2: WO2008/07403A1

### Summary of Invention

### Technical Problem

However, in the device described in PTL 1, it is possible to increase a use length of the medical device in a case where the length of the medical device is too short for the procedure in the target biological lumen. Meanwhile, it is impossible to reduce the use length of the medical device in a case where the length of the medical device is too long for the procedure in the target biological lumen. Accordingly, there is a demand for a device capable of adjusting the use length of the medical device to be longer or shorter according to the procedure in the target biological lumen.

The present invention is made to solve the problems and an object thereof is to provide a medical assistance device capable of adjusting the use length of the medical device according to the procedure in the target biological lumen. Solution to Problem

In order to solve the problems, according to an aspect of the present invention, there is provided a medical assistance device which is connected to a medical device which is inserted into a biological lumen and used in a procedure in the biological lumen, the medical assistance device comprising: a support; a length adjustment unit which is rotatably provided about a support shaft with respect to the support and performs at least one of winding and unwinding of the medical device to adjust a use length of the medical device; an elongated body which is attached to the length adjustment unit and is connected to the medical device, and of which at least one of winding and unwinding is performed with respect to the length adjustment unit; and a connection section which is provided on an end portion of the elongated body and connects the end portion of the elongated body and an end portion of the medical device to each other; a rotation drive unit which is provided in the support and generates a rotation force for rotating the length adjustment unit when a voltage is supplied to the rotation drive unit; wherein the medical assistance device further comprises: a rotation detection unit which detects a rotation angle of the length adjustment unit; and a control unit which receives a signal transmitted from the rotation detection unit and calculates a winding length and an unwinding length of the elongated body from a predetermined position, based on the rotation angle detected by the rotation detection unit.

According to the configuration, the length adjustment unit is provided rotatably about the support shaft with respect to the support. Moreover, the elongated body connected to the medical device is attached to the length adjustment unit. Moreover, the connection section which connects the end portion of the elongated body and the end portion of the medical device to each other is provided on the end portion of the elongated body. That is, the length adjustment unit is connected to the medical device via the connection section and the elongated body. Moreover, the length adjustment unit can perform at least one of the winding and unwinding of the medical device and the elongated body to adjust a use length of the medical device. Accordingly, in a case where the length of the medical device is too short for a procedure in a target biological lumen, the medical assistance device of the present invention unwinds the medical device and an elongated body so that the use length of the medical device can increase. That is, the medical assistance device of the present invention unwinds the elongated body from the length adjustment unit to supplement a too short portion (insufficient portion) of the medical device with the elongated body, and thus, it is possible to increase the use length of the medical device. Meanwhile, in a case where the length of the medical device is too long for the procedure in the target biological lumen, the medical assistance device of the present invention winds the medical device and the elongated body so that the use length of the medical device can be reduced. That is, in the medical assistance device of the present invention, the length adjustment unit winds a too long portion (excess portion) of the medical device, and thus, it is possible to reduce the use length of the medical device. In this way, the medical assistance device of the present invention can adjust the use length of the medical device according to the procedure in the target biological lumen.

Preferably, the length adjustment unit is attachable to or detachable from the support and is replaceable with a new length adjustment unit different from the length adjustment unit.

According to the configuration, the support can be used as a reuse portion. Meanwhile, the length adjustment unit can be used as a disposable portion. That is, after the elongated body connected to the medical device via the connection section is unwound from the support or is inserted into the biological lumen, the elongated body may be cut off by the surgeon or an assistant. Moreover, in a case where the length of the elongated body is shorter than a predetermined length, the surgeon can replace the length adjustment unit with a new length adjustment unit to reuse the medical assistance device.

Preferably, the length adjustment unit has a grip portion which is rotationally operated.

According to the configuration, the length adjustment unit has the grip portion which is rotationally operated. Therefore, even in a case where electricity is not supplied, the surgeon can operate the grip portion so that the length adjustment unit is rotated to adjust the use length of the medical device.

Preferably, the medical assistance device further includes a biasing unit which is connected to the support and the length adjustment unit and biases the length adjustment unit in a direction in which the elongated body is wound.

According to the configuration, the medical assistance device further includes the biasing unit which biases the length adjustment unit in the direction in which the elongated body is wound. Therefore, the length adjustment unit can easily wind the medical device and the elongated body to easily adjust the use length of the medical device.

Preferably, the medical assistance device further includes a lock mechanism which is provided in the support and stops a rotating operation of the length adjustment unit biased by the biasing unit.

According to the configuration, even in a case where the length adjustment unit is biased by the biasing unit, the lock mechanism can stop the rotating operation of the length adjustment unit. Therefore, the surgeon can perform the manipulation with the medical device appropriately bent.

The medical assistance device further includes a rotation drive unit which is provided in the support and generates a rotation force for rotating the length adjustment unit when a voltage is supplied to the rotation drive unit.

According to the configuration, even when the length adjustment unit is not manually operated by the surgeon, a voltage is supplied to the length adjustment unit so that the length adjustment unit can be automatically rotated and can adjust the use length of the medical device.

The medical assistance device further includes a rotation detection unit which detects a rotation angle of the length adjustment unit, and a control unit which receives a signal transmitted from the rotation detection unit and calculates a winding length and an unwinding length of the elongated body from a predetermined position, based on the rotation angle detected by the rotation detection unit.

According to the configuration, even in a state where at least a portion of the medical device and the elongated body is wound around the length adjustment unit, the surgeon can grasp how to take a length of the medical device inserted into a body during the procedure in the biological lumen or a deflection amount (buffer amount) of the medical device based on the length calculated by the control unit.

Preferably, based on the rotation angle detected by the rotation detection unit, the control unit controls the rotation drive unit to unwind the elongated body when the control unit detects a force in a direction in which the elongated body is unwound, and the control unit controls the rotation drive unit to wind the elongated body when the control unit detects a force in a direction in which the elongated body is wound.

According to the configuration, the control unit can control the rotation drive unit to assist a manipulation of the surgeon unwinding or winding the medical device and the elongated body. Thereby, the medical assistance device of the present invention can support the procedure in the biological lumen.

Preferably, the rotation force for rotating the length adjustment unit is a first rotation force, the rotation drive unit for generating the first rotation force is a first rotation drive unit, and a second rotation drive unit which generates a second rotation force for rotating the elongated body about an axis of the elongated body is further provided.

According to the configuration, the second rotation drive unit can generate the second rotation force and rotate the elongated body about the axis of the elongated body. Accordingly, the second rotation drive unit can rotate the medical device connected via the elongated body and the connection section about the axis of the medical device. Accordingly, a choice of the procedure in the biological lumen can be widened. For example, in a case where the medical device is an image diagnosis catheter (for example, IVUS catheter: Intra Vascular Ultra Sound catheter), the second rotation drive unit rotates the medical device about the axis of the medical device in the biological lumen, and thus, an image of a target lesion can be acquired in a circumferential direction. Alternatively, for example, in a case where the medical device is the guide wire, the second rotation drive unit rotates the medical device about the axis of the medical device in the biological lumen, and thus, it is possible to improve selectivity of a blood vessel in a bifurcated portion of the blood vessel.

Preferably, at least one of the support and the length adjustment unit has an introduction portion which is spatially connected to the connection section, and into which another medical device different from the medical device is inserted or a working fluid used at the time of the procedure is fed.

According to the configuration, the surgeon can insert another medical device or feed the working fluid through the introduction portion. For example, in a case where the medical device is a catheter, the surgeon can insert the guide wire into a lumen of the catheter through the introduction portion. Alternatively, for example, in the case where the medical device is the catheter, the surgeon can feed the saline for priming into the lumen of the catheter through the introduction portion.

### Advantageous Effects of Invention

According to the present invention, it is possible to provide a medical assistance device capable of adjusting a use length of a medical device according to a procedure in a target biological lumen.

### Brief Description of Drawings

[Fig. 1] Fig. 1 is a perspective view illustrating a situation before a procedure in a catheter room in which a medical assistance device according to an embodiment of the present invention is installed.
[Fig. 2] Fig. 2 is a perspective view illustrating a situation during the procedure in the catheter room in which the medical assistance device according to the present embodiment is installed.
[Fig. 3] Fig. 3 is a perspective view illustrating a medical assistance device according to a first embodiment of the present invention.
[Fig. 4] Fig. 4 is a perspective view illustrating the medical assistance device according to the first embodiment of the present invention.
[Fig. 5] Fig. 5 is a cross-sectional view illustrating an internal structure of the medical assistance device according to the present embodiment.
[Fig. 6] Fig. 6 is a cross-sectional view illustrating a medical assistance device according to a modification example of the present embodiment.
[Fig. 7] Fig. 7 is a cross-sectional view illustrating the medical assistance device according to the modification example of the present embodiment.
[Fig. 8] Fig. 8 is a perspective view illustrating a medical assistance device according to a second embodiment of the present invention.
[Fig. 9] Fig. 9 is a perspective view illustrating a medical assistance device according to a third embodiment of the present invention.
[Fig. 10] Fig. 10 is a perspective view illustrating a medical assistance device according to a fourth embodiment of the present invention.

### Description of Embodiments

Hereinafter, preferable embodiments of the present invention will be described in detail with reference to the drawings.

Note that since embodiments described below are preferred specific examples of the present invention, although various technically preferable limitations are given, the scope of the present invention is not limited to the embodiments unless otherwise specified in the following descriptions. Moreover, in the drawings, the same reference signs are assigned to the same components, and detailed descriptions thereof are appropriately omitted.

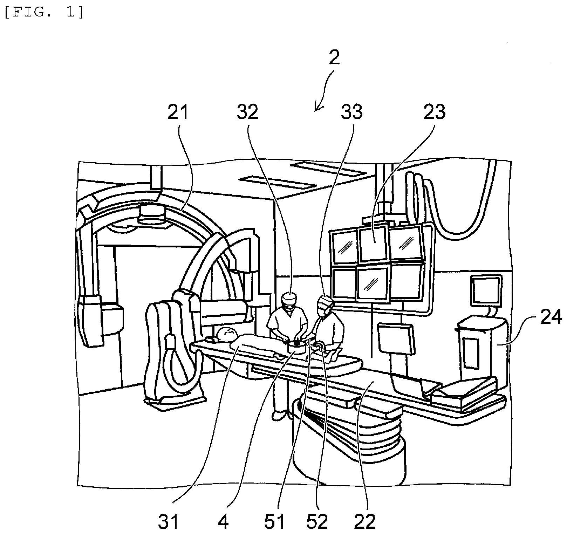

Fig. 1 is a perspective view illustrating a situation before a procedure in a catheter room in which a medical assistance device according to an embodiment of the present invention is installed.

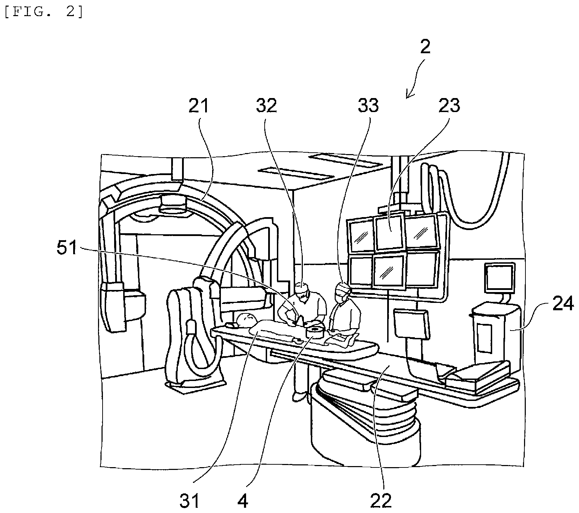

Fig. 2 is a perspective view illustrating a situation during the procedure in the catheter room in which the medical assistance device according to the present embodiment is installed.

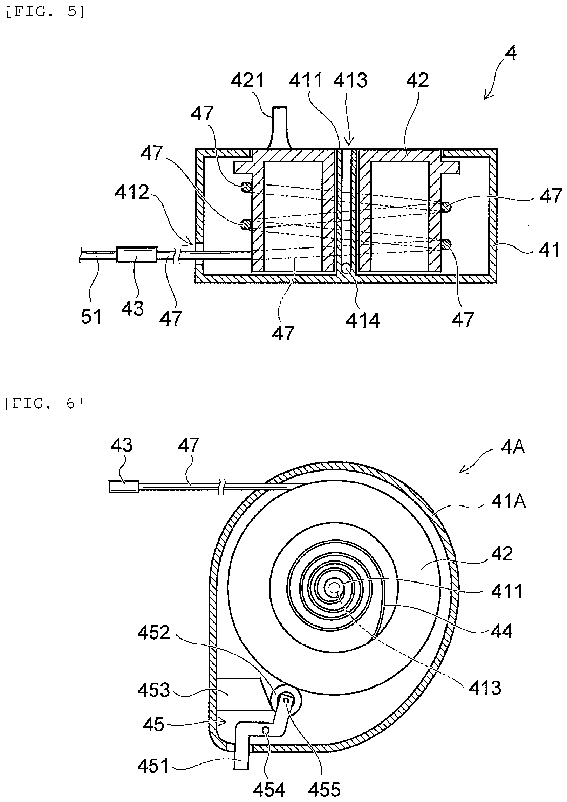

A medical assistance device 4 according to the present embodiment is connected to a medical device such as a catheter or a guide wire used in a procedure in a biological lumen. The medical assistance device 4 can be used regardless of a difference in manipulation in a Percutaneous Coronary Intervention (PCI: coronary angioplasty), an Endovascular Treatment (EVT), or the like and a difference in a puncture site in a Trans-Radial Intervention (TRI), a Trans-Femoral Intervention (TFI), or the like. The same applies to medical assistance devices 4A, 4B, 4C, 4D, 4E, and 4F described later with reference to Figs. 6 to 10.

As illustrated in Figs. 1 and 2, for example, a blood vessel imaging device (angio device) 21, a catheter table 22, a digital imaging device 23, an electrocardiogram (polygraph) 24, a contrast agent automatic injection device (injector, not illustrated), or the like is installed in a catheter room 2 in which a procedure such as a treatment or diagnosis in the biological lumen is performed using a medical device 51 such as the catheter or the guide wire. In addition, a defibrillator, an aorta balloon pumping device, a percutaneous cardiopulmonary assist device, an extracorporeal pacemaker, a ventilator, an emergency drug set, or the like is installed as necessary.

Note that in the catheter room 2 illustrated in Figs. 1 and 2, backs of a surgeon 32 and an assistance 33 face a monitor of the digital imaging device 23 for convenience of explanation. However, actually, in the catheter room 2, the surgeon 32 and the assistance 33 (hereinafter, referred to as a "surgeon 32 or the like" for convenience of explanation) perform the procedure while checking the monitor of the digital imaging device 23. For example, the surgeon 32 or the like moves to a position at which the monitor of the digital imaging device 23 can be checked, and performs the procedure while checking the monitor. Alternatively, for example, the surgeon 32 or the like moves the monitor to the position at which the monitor of the digital imaging device 23 can be checked, and performs the procedure while checking the monitor.

The blood vessel imaging device 21 has an X-ray tube which generates an X-ray and a Flat Panel Detector (FPD) which receives the X-ray transmitted through a patient 31 and converts the X-ray into image information. Moreover, the blood vessel imaging device 21 converts an X-ray image in which the X-ray transmitted through the patient 31 from the X-ray tube is attenuated by an anatomical structure of the patient 31 and a contrast agent into a digital image signal by the Flat Panel Detector and transmits the converted digital image signal to the digital imaging device 23. The catheter table 22 is a table on which the patient 31 lays down, and a top plate portion of catheter table 22 can move up and down and move in the front-rear and left-right directions in a state where the patient 31 lays down. The digital imaging device 23 receives the X-ray image transmitted from the blood vessel imaging device 21, digitally records the X-ray image, and automatically reproduces the X-ray image on a reference monitor. Further, the digital imaging device 23 can perform slow reproduction and stillness of the X-ray image or zoom-in of a lesion area.

As described above, various devices are installed in the catheter room 2. Accordingly, a space in which the surgeon 32, the assistance 33, or the like can be active in the catheter room 2 is not so large and is limited. As illustrated in Fig. 1, the surgeon 32 or the like pulls out a medical device 51 such as a catheter from a medical device holder 52 such as a catheter holder in a limited narrow space of the catheter room 2 and prepares (starts-up or sets-up) a procedure in a biological lumen. Further, as illustrated in Fig. 2, the surgeon 32 or the like operates the medical device 51 in the limited space of the catheter room 2 or operates the blood vessel imaging device 21, the catheter table 22, or the like to perform the procedure in the biological lumen.

For example, when the Trans-Radial Intervention is adopted in a procedure in a biological lumen (peripheral blood vessel) of a lower limb, a length of the medical device 51 may not only be longer than the height of the patient 31, the surgeon 32, or the like, but may also be longer than the catheter table 22 of which an entire length in a longitudinal direction is about 330 cm. Moreover, a distance from the puncture site to a procedure site is changed depending on an age, a height, or a body type of a patient. Accordingly, the length of the existing medical device 51 may be too long or too short for a procedure in a target biological lumen. That is, the length of the existing medical device 51 may not match the procedure in the target biological lumen.

Meanwhile, the medical assistance device 4 according to the present embodiment performs at least one of winding and unwinding of the medical device 51 so that a use length of the medical device 51 can be adjusted. In Figs. 1 and 2, the medical assistance device 4 is placed at or near a crotch or thigh of the patient 31, but may be placed on the catheter table 22. For example, the medical assistance device 4 may be fixed to a predetermined location of the catheter table 22. Alternatively, the medical assistance device 4 may be freely detachably attached to the predetermined location of the catheter table 22. An installation form of the medical assistance device 4 is not particularly limited.

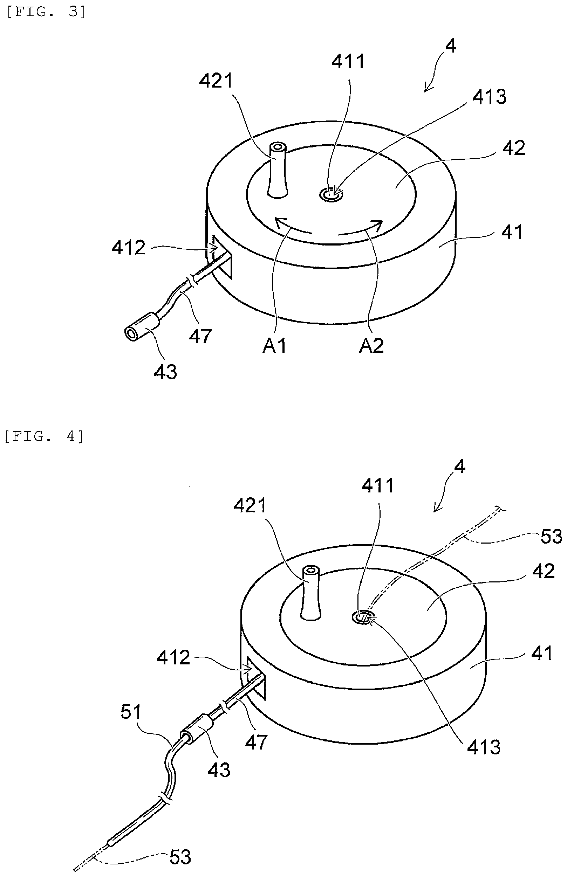

According to the present embodiment, in a case where the length of the medical device 51 is too short for the procedure in the target biological lumen, the medical assistance device 4 unwinds the medical device 51 and an elongated body 47 (refer to 3) so that the use length of the medical device 51 can increase. That is, the medical assistance device 4 unwinds the elongated body 47 from a length adjustment unit 42 (refer to Fig. 3) to supplement a too short portion (insufficient portion) of the medical device 51 with the elongated body 47, and thus, it is possible to increase the use length of the medical device 51. Meanwhile, in a case where the length of the medical device 51 is too long for the procedure in the target biological lumen, the medical assistance device 4 winds the medical device 51 and the elongated body 47 so that the use length of the medical device 51 can be reduced. That is, in the medical assistance device 4, the length adjustment unit 42 winds a too long portion (excess portion) of the medical device 51, and thus, it is possible to reduce the use length of the medical device 51. In this way, the medical assistance device 4 can adjust the use length of the medical device 51 according to the procedure in the target biological lumen. Hereinafter, the medical assistance device 4 according to the present embodiment will be described in detail with reference to the drawings.

Figs. 3 and 4 are perspective views illustrating a medical assistance device according to a first embodiment of the present invention.

Fig. 5 is a cross-sectional view illustrating an internal structure of the medical assistance device according to the present embodiment.

Note that Fig. 4 illustrates a state in which another medical device 53 (for example, guide wire) is inserted into the medical device 51 (for example, catheter) through an introduction portion 413.

The medical assistance device 4 according to the present embodiment includes a support 41, a length adjustment unit 42, an elongated body 47, and a connection section 43.

The support 41 is provided as a main body of the medical assistance device 4 and has a support shaft 411. The support 41 illustrated in Figs. 3 to 5 has a housing structure in which an internal space is provided. However, the support 41 of the present embodiment is not limited to having an internal space, and the length adjustment unit 42 may be exposed outside the support 41 as long as the length adjustment unit 42 is rotatably supported. The support 41 has an opening portion 412. In the examples illustrated in Figs. 3 to 5, the elongated body 47 and the connection section 43 are disposed outside the support 41 through the opening portion 412 of the support 41. Note that in a case where most of the length adjustment unit 42 is exposed to the outside of the support 41, the support 41 needs not necessarily have the opening portion 412.

The introduction portion 413 is provided in one end portion of the support shaft 411. A derivation portion 414 is provided in the other end portion of the support shaft 411. The introduction portion 413 is spatially connected to the elongated body 47 and the connection section 43 via an inside of the support shaft 411 and a derivation portion 414. Accordingly, as illustrated in Fig. 4, the surgeon 32 can insert the medical device 53 such as the guide wire into the elongated body 47 and the connection section 43 through the introduction portion 413 of the support shaft 411, the inside of the support shaft 411, or the derivation portion 414 of the support shaft 411 or can feed a saline for priming into the elongated body 47 and the connection section 43. Note that the introduction portion 413 and the derivation potion 414 are not limited to being provided in the support shaft 411, and may be provided in other portions of the length adjustment unit 42 or in the support 41.

The length adjustment unit 42 is provided to be rotatable about the support shaft 411 of the support 41 with respect to the support 41, as indicated by arrows A1 and A2 illustrated in Fig. 3. Note that the support shaft 411 is not limited to being provided in the support 41, and may be provided in the length adjustment unit 42. That is, the length adjustment unit 42 may be provided rotatably with respect to the support 41 about the support shaft 411 provided in at least one of the support 41 and the length adjustment unit 42. The same applies to the medical assistance devices 4A, 4B, 4C, 4D, 4E, and 4F described later with reference to Figs. 6 to 10.

The length adjustment unit 42 has a grip portion 421. The grip portion 421 projects outward from a surface of the length adjustment unit 42 and assists a rotating operation of the length adjustment unit 42. That is, the surgeon 32 or the like can hold and operate the grip portion 421 of the length adjustment unit 42 to easily rotate the length adjustment unit 42. Note that the grip portion 421 need not necessarily have a projection portion, and may be a concave portion into which a finger can be inserted.

The length adjustment unit 42 is attachable to or detachable from the support 41. For example, the support 41 includes a base portion and a lid portion which is attachable to and detachable from the base portion. When the lid portion of the support 41 is removed from the base portion of the support 41, the length adjustment unit 42 supported by the support 41 can be removed from the support 41. The length adjustment unit 42 is removed from the support 41, and thus, a new length adjustment unit can be replaced. That is, the support 41 can be used as a reuse portion. Meanwhile, the length adjustment unit 42 can be used as a disposable portion.

The elongated body 47 is attached to the length adjustment unit 42. Specifically, one end portion of the elongated body 47 is connected to an outer peripheral portion of the length adjustment unit 42. Accordingly, when the length adjustment unit 42 rotates, the elongated body 47 is wound around the outer peripheral portion of the length adjustment unit 42 or is unwound from the length adjustment unit 42. The other end portion of the elongated body 47 is connected to the connection section 43. The elongated body 47 is a catheter or a wire for adjusting the use length of the medical device 51, and for example, in a case where the length of the medical device 51 is too short for the procedure in the target biological lumen, the elongated body 47 can supplement the length of the medical device 51.

A material of the elongated body 47 may be the same as a material of the medical device 51 or may be different from the material of the medical device 51. For example, in a case where the medical device 51 connected to the connection section 43 is a catheter, the elongated body 47 may be a wire or may be a tube having the same lumen as the catheter. In addition, for example, in a case where the medical device 51 connected to the connection section 43 is a guide wire, the elongated body 47 may be a tube having a lumen or may be a wire similar to the medical device 51.

The connection section 43 is provided in the other end portion of the elongated body 47. Specifically, one end portion of the connection section 43 is connected to the other end portion of the elongated body 47. Therefore, when the length adjustment unit 42 rotates, the connection section 43 is wound around the outer peripheral portion of the length adjustment unit 42 together with the elongated body 47 or is unwound from the length adjustment unit 42. The other end portion of the connection section 43 is connected to a proximal portion of the medical device 51. That is, the connection section 43 connects the other end portion of the elongated body 47 and the proximal portion of the medical device 51 to each other. In other words, the elongated body 47 is connected to the medical device 51 via the connection section 43. Note that in the present specification, a side inserted into a lumen of a living body is referred to as a "distal end" or a "distal side", and a hand side operated by the surgeon is referred to as a "proximal end" or a "proximal side".

A structure and a form of the connection section 43 are not particularly limited. For example, an outer diameter of the connection section 43 may be the same as an outer diameter of a proximal portion of the connected medical device 51 or may be larger or smaller than the outer diameter of the proximal portion of the medical device 51.

Next, an operation of the medical assistance device 4 according to the present embodiment will be described.

As illustrated in Fig. 1, the surgeon 32 or the like pulls out the proximal portion of the medical device 51 from the medical device holder 52 and connects the medical device 51 to the elongated body 47 via the connection section 43 of the medical assistance device 4. Then, the surgeon 32 or the like holds the grip portion 421 of the length adjustment unit 42 and rotates the length adjustment unit 42 in a direction of the arrow A1 illustrated in Fig. 3. Accordingly, the elongated body 47 is wound around the outer peripheral portion of the length adjustment unit 42. Therefore, as illustrated in Fig. 5, the medical device 51 is pulled out from the medical device holder 52, and is connected to the elongated body 47, which is wound around the outer peripheral portion of the length adjustment unit 42, via the connection section 43.

In a case where the length of the medical device 51 is too short for the procedure in the target biological lumen, the surgeon 32 or the like pulls the medical device 51 so that the medical device 51 and the elongated body 47 can be unwound from the support 41. Meanwhile, in a case where the length of the medical device 51 is too long for the procedure in the target biological lumen, the surgeon 32 or the like rotates the length adjustment unit 42 in the direction of the arrow A1 illustrated in Fig. 3 using the grip portion 421 of the length adjustment unit 42 so that the too long portion (excess portion) of the medical device 51 can be wound around the outer peripheral portion of the length adjustment unit 42.

According to the present embodiment, in the case where the length of the medical device 51 is too short for the procedure in the target biological lumen, the medical assistance device 4 can unwind the medical device 51 and the elongated body 47 to increase the use length of the medical device 51. That is, the medical assistance device 4 can unwind the elongated body 47 from the length adjustment unit 42 and supplement the too short portion (insufficient portion) of the medical device 51 with the elongated body 47 to increase the use length of the medical device 51. Meanwhile, in the case the length of the medical device 51 is too long for the procedure in the target biological lumen, the medical assistance device 4 can wind the medical device 51 and the elongated body 47 to reduce the use length of the medical device 51. That is, the medical assistance device 4 can wind the too long portion (excess portion) of the medical device 51 around the length adjustment unit 42 to reduce the use length of the medical device 51. In this way, the medical assistance device 4 can adjust the use length of the medical device 51 according to the procedure in the target biological lumen.

Moreover, as described above, the length adjustment unit 42 is provided so as to be attachable to and detachable from the support 41, and can be replaced with a new length adjustment unit different from the length adjustment unit 42. Meanwhile, the length adjustment unit 42 can be used as a disposable portion. That is, after the elongated body 47 connected to the medical device 51 via the connection section 43 is unwound from the support 41 or is inserted into the biological lumen, the elongated body 47 may be cut off by the surgeon 32 or the like. Moreover, in a case where the length of the elongated body 47 is shorter than a predetermined length, the surgeon 32 or the like can replace the length adjustment unit 42 with a new length adjustment unit to reuse the medical assistance device 4.

Moreover, the length adjustment unit 42 has the rotationally operable grip portion 421. Accordingly, in a case where electricity is not provided, the surgeon 32 or the like can operate the grip portion 421 to rotate the length adjustment unit 42 and adjust the use length of the medical device 51. For example, at the time of a disaster, the surgeon 32 or the like winds the medical device 51 and the elongated body 47 around the length adjustment unit 421 and performs at least one of winding and unwinding of the medical device 51 and the elongated body 47 at a disaster site. Accordingly, it is possible to adjust the use length of the medical device 51.

Moreover, as described above, one end portion of the support shaft 411 include the introduction portion 413 which is spatially connected to the elongated body 47 and the connection section 43. Accordingly, the surgeon 32 or the like can insert another medical device 53 or feed a working fluid through the introduction portion 413. For example, the "working fluid" in the present specification includes a gas such as air, helium gas, CO₂ gas, and O₂ gas, or a liquid such as a contrast agent, a drug solution, a coating agent, and a saline. For example, in a case where the medical device 51 is a catheter, the surgeon 32 or the like can insert the guide wire into a lumen of the catheter through the introduction portion 413. Alternatively, for example, in the case where the medical device 51 is the catheter, the surgeon 32 or the like can feed the saline for priming into the lumen of the catheter through the introduction portion 413.

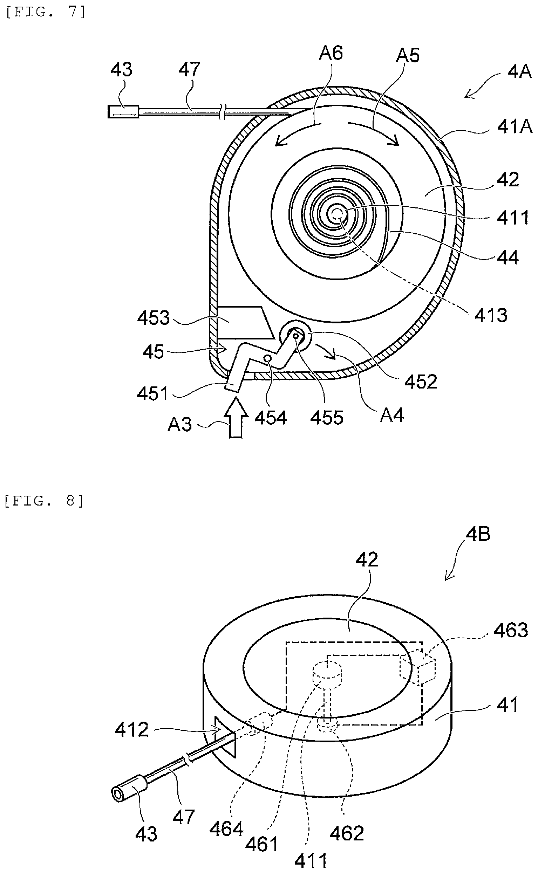

Note that in the medical assistance device 4 illustrated in Figs. 3 to 5, the length adjustment unit 42 is provided rotatably with respect to the support 41 about the support shaft 411 extending in a vertical direction. However, an installation form of the length adjustment unit 42 is not limited to this. For example, the length adjustment unit 42 may be provided rotatably with respect to the support 41 about the support shaft 411 extending in a horizontal direction. The same applies to the medical device 4A described later with reference to Figs. 6 and 7, and the medical assistance device 4B described later with reference to Fig. 8.

Next, a medical assistance device according to a modification example of the present embodiment will be described.

Note that in a case where components of the medical assistance device 4A according to the present modification example are the same as the components of the medical assistance device 4 according to the present embodiment described above with reference to Figs. 3 to 5, repeated descriptions are appropriately omitted, and differences therebetween will be mainly described.

Figs. 6 and 7 are cross-sectional views illustrating the medical assistance device according to the modification example of the present embodiment.

Note that Fig. 6 is a cross-sectional view illustrating a state where locking of the length adjustment unit is set. Fig. 7 is a cross-sectional view illustrating a state where the locking of the length adjustment unit is released.

The medical assistance device 4A according to the present modification example further includes a biasing unit 44 and a lock mechanism 45 as compared with the medical assistance device 4 described above with reference to Figs. 3 to 5. The biasing unit 44 is connected to the support shaft 411 of a support 41A and the length adjustment unit 42, and biases the length adjustment unit 42 in a direction (a direction of an arrow A5 illustrated in Fig. 7) in which the medical device 51 and the elongated body 47 are wound. Accordingly, in a case where the surgeon 32 or the like unwinds the medical device 51 connected to the connection section 43 from the support 41A, the surgeon 32 pulls the medical device 51 against a biasing force of the biasing unit 44. For example, the biasing unit 44 includes a mainspring, a leaf spring, or the like.

The lock mechanism 45 includes an operation unit 451, a roller 452, and a pressing portion 453. The operation unit 451 is provided so as to be rotatable about a first shaft 454 provided in the support 41A with respect to the support 41A. Moreover, the operation unit 451 is biased in a direction in which the roller 452 faces the pressing portion 453. As a biasing unit for biasing the operation unit 451 in the direction in which the roller 452 faces the pressing portion 453, for example, a torsion coil spring or the like can be used. The roller 452 is provided in a distal portion of the operation unit 451 so as to be rotatable about a second shaft 455 provided in the operation unit 451 with respect to the operation unit 451. The pressing portion 453 is fixed to an inner surface of the support 41A.

As illustrated in Fig. 7, a gap between the pressing portion 453 and an outer peripheral portion of the length adjustment unit 42 is narrowed in the biasing direction (the winding direction of the medical device 51 and the elongated body 47: the direction of the arrow A5) of the biasing unit 44. That is, the gap between the pressing portion 453 and the outer peripheral portion of the length adjustment unit 42 has a wedge shape. Then, in the biasing direction of the biasing unit 44, the gap between the pressing portion 453 and the outer peripheral portion of the length adjustment unit 42 is shorter than an outer diameter of the roller 452.

As illustrated in Fig. 6, in a state where the roller 452 is interposed between the pressing portion 453 and the length adjustment unit 42, the locking of the length adjustment unit 42 is set. That is, in the state illustrated in Fig. 6, the lock mechanism 45 stops the rotating operation of the length adjustment unit 42 biased by the biasing unit 44. When the operation unit 451 is pushed in a direction of an arrow A3 illustrated in Fig. 7 from the state illustrated in Fig. 6, the operation unit 451 rotates about the first shaft 454 in a direction of an arrow A4 illustrated in Fig. 7. Then, the roller 452 moves away from the pressing portion 453 while rotating about the second shaft 455. Thereby, the locking of the length adjustment unit 42 is released. In the state illustrated in Fig. 7, the length adjustment unit 42 rotates in the direction (the direction of the arrow A5 illustrated in Fig. 7) in which the medical device 51 and the elongated body 47 are wound by the biasing force applied from the biasing unit 44.

As described above, the operation unit 451 is biased in the direction in which the roller 452 faces the pressing portion 453. Therefore, when the force in the direction of the arrow A3 illustrated in Fig. 7 is released from the operation unit 451, the operation unit 451 rotates about the first shaft 454 in a direction opposite to the arrow A4 illustrated in Fig. 7. Then, the roller 452 is interposed again between the pressing portion 453 and the outer peripheral portion of the length adjustment unit 42. Accordingly, the locking of the length adjustment unit 42 is set again.

Next, an operation of the medical assistance device 4A according to the modification example will be described.

As illustrated in Fig. 1, the surgeon 32 or the like pulls out the proximal portion of the medical device 51 from the medical device holder 52 and connects the medical device 51 to the connection section 43 of the medical assistance device 4. Then, when the surgeon 32 or the like pushes the operation unit 451 in the direction of the arrow A3 illustrated in Fig. 7, the length adjustment unit 42 rotates in the direction of the arrow A5 illustrated in Fig. 7 by the biasing force applied from the biasing unit 44. Therefore, the medical device 51 is pulled out from the medical device holder 52. Moreover, the medical device 51 and the elongated body 47 are wound around the outer peripheral portion of the length adjustment unit 42 (refer to Fig. 5) . When the surgeon 32 or the like releases the force pushing the operation unit 451, the roller 452 is interposed between the pressing portion 453 and the outer peripheral portion of the length adjustment unit 42, and thus, the locking of the length adjustment unit 42 is set.

In the case where the length of the medical device 51 is too short for the procedure in the target biological lumen, the surgeon 32 or the like pulls the medical device 51 so that the medical device 51 and the elongated body 47 can be unwound from the support 41. When the surgeon 32 or the like pulls the medical device 51 against the biasing force of the biasing unit 44, the operation unit 451 is rotated about the first shaft 454 in the direction of the arrow A4 illustrated in Fig. 7 by a friction force generated between the roller 452 and the outer peripheral portion of the length adjustment unit 42. Then, the roller 452 moves away from the pressing portion 453 while rotating about the second shaft 455. Thereby, the locking of the length adjustment unit 42 is released, and the medical device 51 and the elongated body 47 are unwound from the support 41A.

Meanwhile, in the case where the length of the medical device 51 is too long for the procedure in the biological lumen, the surgeon 32 or the like pulls the medical device 51 while maintaining a state where at least a portion of the medical device 51 is wound around the length adjustment unit 42. That is, the surgeon 32 or the like pulls the medical device 51 while maintain a state where the too long portion (excess portion) of the medical device 51 is wound around the outer peripheral portion of the length adjustment unit 42. Accordingly, as described above, the locking of the length adjustment unit 42 is released, and the medical device 51 and the elongated body 47 are unwound from the support 41A in a state where at least a portion of the medical device 51 is wound around the length adjustment unit 42.

When the surgeon 32 or the like unwinds the medical device 51 from the support 41A by a required length thereof and releases the pulling force of the medical device 51, the length adjustment unit 42 tries to rotate in the direction of the arrow A5 illustrated in Fig. 7 by the biasing force given from the biasing unit 44. In this case, the operation unit 451 is biased in the direction in which the roller 452 faces the pressing portion 453. Accordingly, the operation unit 451 rotates about the first shaft 454 in the direction opposite to the arrow A4 illustrated in Fig. 7. Then, the roller 452 is interposed again between the pressing portion 453 and the outer peripheral portion of the length adjustment unit 42. Thereby, the locking of the length adjustment unit 42 is set again.

According to the medical assistance device 4A of the present modification example, the biasing unit 44 which biases the length adjustment unit 42 in the direction in which the elongated body 47 is wound is provided. Accordingly, the length adjustment unit 42 can easily wind the medical device 51 and the elongated body 47, easily adjusting the use length of the medical device 51. Further, even in a case where the length adjustment unit 42 is biased by the biasing unit 44, the lock mechanism 45 can stop the rotating operation of the length adjustment unit 42. Therefore, the surgeon 32 or the like can perform the manipulation with the medical device 51 appropriately bent.

Note that in the present modification, the case where the lock mechanism 45 includes the operation unit 451, the roller 452, and the pressing portion 453 is described as an example. However, the lock mechanism of the present modification example is not limited to the example illustrated in Figs. 6 and 7. For example, an automatic winding type mechanism may be used, in which when the surgeon 32 or the like pulls the medical device 51, the locking of the length adjustment unit 42 is set, and when the surgeon 32 or the like pulls the medical device 51 again, the locking of the length adjustment unit 42 is released and the length adjustment unit 42 winds the medical device 51 and the elongated body 47.

Next, a medical assistance device according to a second embodiment of the present invention will be described.

Note that in a case where components of the medical assistance device 4B according to the second embodiment are the same as the components of the medical assistance device 4 according to the first embodiment described above with reference to Figs. 3 to 5 and the components of the medical assistance device 4A according to the modification example with reference to Figs. 6 and 7, repeated descriptions are appropriately omitted, and hereinafter, differences therebetween will be mainly described.

Fig. 8 is a perspective view illustrating the medical assistance device according to the second embodiment of the present invention.

The medical assistance device 4B according to the present embodiment includes the support 41, the length adjustment unit 42, the connection section 43, a first rotation drive unit 461, a rotation detection unit 462, a control unit 463, and a second rotation drive unit 464. The grip portion 421 described above with reference to Figs. 3 to 5 may or may not be provided. In an example illustrated in Fig. 8, the grip portion 421 is not provided.

The first rotation drive unit 461 is provided in the support 41 and connected to the length adjustment unit 42. When a voltage is supplied to the first rotation drive unit 461, the first rotation drive unit 461 generates a rotation force (first rotation force) for rotating the length adjustment unit 42. The rotation force generated by the first rotation drive unit 461 of the present embodiment corresponds to a "first rotation force" of the present invention. For example, the first rotation drive unit 461 is a motor and is connected to the length adjustment unit 42 via a predetermined gear train. The first rotation drive unit 461 is driven based on a control signal transmitted from the control unit 463 to rotate the length adjustment unit 42. In a case where the first rotation drive unit 461 is a stepping motor, the position of the length adjustment unit 42 can be determined with high accuracy.

The rotation detection unit 462 is provided in the support 41 and detects a rotation angle of the length adjustment unit 42. The rotation detection unit 462 transmits a detection signal regarding the detected rotation angle of the length adjustment unit 42 to the control unit 463. For example, the rotation detection unit 462 includes a rotary encoder, a potentiometer, or the like. Alternatively, as the rotation detection unit 462, a pressure sensor or the like for detecting a torque generated in the length adjustment unit 42 may be used.

The control unit 463 transmits the control signal to the first rotation drive unit 461 and the second rotation drive unit 464 to control operations of the first rotation drive unit 461 and the second rotation drive unit 464. In addition, the control unit 463 receives the detection signal transmitted from the rotation detection unit 462 to execute a predetermined process.

Specifically, based on the rotation angle of the length adjustment unit 42 detected by the rotation detection unit 462, the control unit 463 calculates a length of the elongated body 47 wound from a predetermined position and a length of the unwound elongated body 47. According to this, even in a state where at least a portion of the medical device 51 and the elongated body 47 is wound around the length adjustment unit 42, the surgeon 32 or the like can grasp how to take the length of the medical device 51 inserted into the body during the procedure in the biological lumen or a deflection amount (buffer amount) of the medical device 51 based on the length calculated by the control unit 463.

Moreover, when the control unit 463 detects a force in the direction (the direction of the arrow A2 illustrated in Fig. 3) in which the elongated body 47 is unwound based on the rotation angle of the length adjustment unit 42 detected by the rotation detection unit 462, the control unit 463 controls the first rotation drive unit 461 to unwind the elongated body 47. That is, the control unit 463 controls the first rotation drive unit 461 to rotate the length adjustment unit 42 in the direction (the direction of the arrow A2 illustrated in Fig. 3) in which the elongated body 47 is unwound. Meanwhile, when the control unit 463 detects a force in the direction (the direction of the arrow A1 illustrated in Fig. 3) in which the elongated body 47 is wound based on the rotation angle of the length adjustment unit 42 detected by the rotation detection unit 462, the control unit 463 controls the first rotation drive unit 461 to wind the elongated body 47. That is, the control unit 463 controls the first rotation drive unit 461 to rotate the length adjustment unit 42 in the direction (the direction of the arrow A1 illustrated in Fig. 3) in which the elongated body 47 is wound.

According to this, the control unit 463 can control the first rotation drive unit 461 to assist a manipulation or an operation of the surgeon 32 or the like unwinding or winding the medical device 51 and the elongated body 47. Thereby, the medical assistance device 4B according to the present embodiment can support the procedure in the biological lumen.

The second rotation drive unit 464 is provided in the support 41 and is connected to the elongated body 47. When a voltage is supplied to the second rotation drive unit 464, the second rotation drive unit 464 generates a rotation force (second rotation force) for rotating the elongated body 47 about an axis of the elongated body 47. That is, the second rotation drive unit 464 generates the rotation force (second rotation force) for rotating the elongated body 47 and the medical device 51 connected via the connection section 43 about the axis of the medical device 51. The rotation force generated by the second rotation drive unit 464 of the present embodiment corresponds to a "second rotation force" of the present invention. For example, the second rotation drive unit 464 is a motor and is connected to the elongated body 47 via a predetermined gear train. The second rotation drive unit 464 is driven based on a control signal transmitted from the control unit 463 to rotate the medical device 51 via the elongated body 47 and the connection section 43. In a case where the second rotation drive unit 464 is a stepping motor, the rotation positions of the elongated body 47, the connection section 43, and the medical device 51 can be determined with high accuracy.

According to the medical assistance device 4B of the present embodiment, even when the length adjustment unit 42 is not manually operated by the surgeon 32 or the like, a voltage is supplied to the length adjustment unit 42 so that the length adjustment unit 42 can be automatically rotated and can adjust the use length of the medical device 51.

Moreover, as described above, the second rotation drive unit 464 can rotate the medical device 51 about the axis of the medical device 51 connected via the elongated body 47 and the connection section 43. Accordingly, a choice of the procedure in the biological lumen can be widened. For example, in a case where the medical device 51 is an image diagnosis catheter (for example, IVUS catheter: Intra Vascular Ultra Sound catheter), the second rotation drive unit 464 rotates the medical device 51 about the axis of the medical device 51 in the biological lumen, and thus, an image of a target lesion can be acquired in a circumferential direction. Alternatively, for example, in a case where the medical device 51 is the guide wire, the second rotation drive unit 464 rotates the medical device 51 about the axis of the medical device 51 in the biological lumen, and thus, it is possible to improve selectivity of a blood vessel in a bifurcated portion of the blood vessel. Further, the same effects as those of the medical assistance device 4 according to the first embodiment described above with reference to Figs. 3 to 5 can be obtained.

Note that the medical assistance device 4B according to the present embodiment may be connected to an external drive apparatus which holds the medical assistance device 4B and drives the medical assistance device 4B. For example, the external drive apparatus can be interlocked with the medical assistance device 4B to perform forward and pull-back operations of the medical device 51 or rotate the medical device 51 about the axis of the medical device 51.

In the medical assistance device 4B according to the present embodiment, the grip portion 421 described above with reference to Figs. 3 to 5 may be provided. In a case where the grip portion 421 is provided, the surgeon 32 or the like can rotate the length adjustment unit 42 manually. Further, in this case, a switching unit may be provided, which can switch between a manual mode in which the length adjustment unit 42 is manually rotated by the grip portion 421 and an electric mode in which the length adjustment unit 42 is electrically driven by the first rotation drive unit 461.

Next, a medical assistance device according to a third embodiment of the present invention will be described.

Note that in a case where components of the medical assistance device 4C according to the third embodiment are the same as the components of the medical assistance device 4 according to the first embodiment described above with reference to Figs. 3 to 5 and the components of the medical assistance device 4B according to the embodiment described above with reference to Fig. 8, repeated descriptions are appropriately omitted, and hereinafter, differences therebetween will be mainly described.

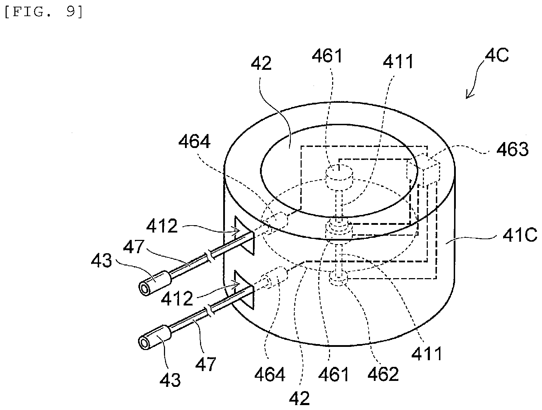

Fig. 9 is a perspective view illustrating the medical assistance device according to the third embodiment of the present invention.

The medical assistance device 4C according to the present embodiment includes a support 41C, two length adjustment units 42, two connection sections 43, two first rotation drive units 461, two rotation detection units 462, the control unit 463, two second rotation drive units 464, and two elongated bodies 47. The two length adjustment units 42 can be rotated independently of each other. For example, the two length adjustment units 42 are provided rotatably with respect to the support 41C about two support shafts 411 disposed coaxially with each other. Alternatively, for example, the two length adjustment units 42 may be provided rotatably with respect to the support 41C about one support shaft 411 common to the two length adjustment units 42.

One first rotation drive unit 461 is provided in the support 41C and connected to one length adjustment unit 42. When a voltage is supplied to the one first rotation drive unit 461, the first rotation drive unit 461 generates a rotation force (first rotation force) for rotating the one length adjustment unit 42. One rotation detection unit 462 is provided in the support 41C and detects a rotation angle of the one length adjustment unit 42. One second rotation drive unit 464 is provided in the support 41C and is connected to one elongated body 47. When a voltage is supplied to the one second rotation drive unit 464, the one second rotation drive unit 464 generates a rotation force (second rotation force) for rotating the one elongated body 47 about the axis of the one elongated body 47. That is, the one second rotation drive unit 464 generates the rotation force (second rotation force) for rotating the medical device 51 connected via the one elongated body 47 and the one connection section 43 about the axis of the medical device 51.

The other first rotation drive unit 461 is provided in the support 41C and connected to the other length adjustment unit 42. When a voltage is supplied to the other first rotation drive unit 461, the other first rotation drive unit 461 generates a rotation force (first rotation force) for rotating the other length adjustment unit 42. The other rotation detection unit 462 is provided in the support 41C and detects a rotation angle of the other length adjustment unit 42. The other second rotation drive unit 464 is provided in the support 41C and is connected to the other elongated body 47. When a voltage is supplied to the other second rotation drive unit 464, the other second rotation drive unit 464 generates a rotation force (second rotation force) for rotating the elongated body 47 about the axis of the other elongated body 47. That is, the other second rotation drive unit 464 generates the rotation force (second rotation force) for rotating the medical device 51 connected via the other elongated body 47 and the other connection section 43 about the axis of the medical device 51.

According to the present embodiment, the two length adjustment units 42 can adjust the use lengths of the medical devices 51 of different types from each other. For example, in the medical assistance device 4C illustrated in Fig. 9, the one length adjustment unit 42 can adjust the use length of the catheter. In addition, the other length adjustment unit 42 can adjust the use length of the guide wire. Accordingly, the choice of the procedure in the biological lumen can be widened.

As described above, the two length adjustment units 42 can be rotated independently of each other. Accordingly, the control unit 463 individually controls the two first rotation drive units 461 so that the use lengths of the two medical devices 51 can be controlled individually. In addition, the control unit 463 individually controls the two second rotation drive units 464 so that an angle at which each of the two medical devices 51 rotates about the center of the axis can be controlled individually.

Note that in the medical assistance device 4C according to the present embodiment, the two length adjustment units 42 may be fixed to each other. In this case, the two length adjustment units 42 can be rotated in conjunction with each other. Moreover, in this case, one first rotation drive unit 461 and one rotation detection unit 462 may be provided. According to this, the control unit 463 can match the use lengths of the two medical devices 51 of different types from each other. Note that the number of the length adjustment unit 42 of the present embodiment is not limited to two and may be three or more.

Moreover, in the medical assistance device 4C illustrated in Fig. 9, the two length adjustment units 42 are provided rotatably with respect to the support 41C about the support shaft 411 extending in the vertical direction. However, installation forms of the two length adjustment units 42 are not limited to this. For example, the two length adjustment units 42 may be provided rotatably with respect to the support 41C about the support shaft 411 extending in the horizontal direction. In this case, it is preferable that the two length adjustment units 42 are disposed to be arranged in the horizontal direction, not in an up-down direction.

Next, a medical assistance device according to a fourth embodiment of the present invention will be described.

Note that in a case where components of the medical assistance device D according to the fourth embodiment are the same as the components of the medical assistance device 4 according to the first embodiment described above with reference to Figs. 3 to 5, the components of the medical assistance device 4B according to the second embodiment described above with reference to Fig. 8, and the components of the medical assistance device 4C according to the third embodiment described above with reference to Fig. 9, repeated descriptions are appropriately omitted, and hereinafter, differences therebetween will be mainly described.

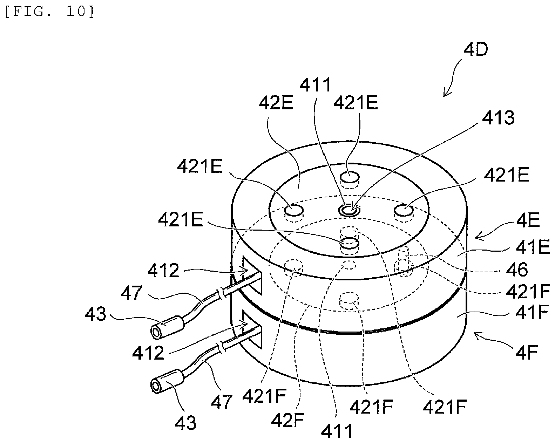

Fig. 10 is a perspective view illustrating the medical assistance device according to the fourth embodiment of the present invention.

The medical assistance device 4D according to the present embodiment has a structure in which a plurality of medical assistance devices are stacked and coupled to each other. In an example illustrated in Fig. 9, two medical assistance devices 4E and 4F are stacked along an axial direction of the support shaft 411 and coupled to each other. The two medical assistance devices 4E and 4F are attachable to and detachable from each other. Note that in the present embodiment, the number of medical assistance devices to be stacked is not limited to two, and may be three or more.

The medical assistance device 4E disposed at a relatively upper stage includes a support 41E, a length adjustment unit 42E, the elongated body 47, and the connection section 43. The length adjustment unit 42E includes concave portions 421E which are provided on an upper surface of the length adjustment unit 42E. he surgeon 32 or the like inserts a finger into the concave portion 421E of the length adjustment unit 42E so that the length adjustment unit 42E can rotate about the support shaft 411 with respect to the support 41E. A coupling portion 46 protruding downward is provided on a lower surface of the length adjustment unit 42E. The coupling portion 46 is inserted into concave portions 421F of the medical assistance device 4F which is disposed at a relatively lower stage.

The lower-stage medical assistance device 4F includes a support 41F, a length adjustment unit 42F, the elongated body 47, and the connection section 43. The length adjustment unit 42F includes the concave portions 421F which are provided on an upper surface of the length adjustment unit 42F. The coupling portion 46 which is provided in the length adjustment unit 42E of the upper-stage medical assistance device 4E is inserted into the concave portion 421F.

The coupling portion 46 is fixed to the lower surface of the length adjustment unit 42E, and when the length adjustment unit 42E rotates, the coupling portion 46 rotates about the support shaft 411 with respect to the support 41E together with the length adjustment unit 42E. Accordingly, when the surgeon 32 or the like inserts a finger into the concave portion 421E of the upper-stage medical assistance device 4E and rotates the length adjustment unit 42E, the length adjustment unit 42F of the lower-stage medical assistance device 4F receives a force from the coupling portion 46 inserted into the concave portion 421F and rotates about the support shaft 411 together with the length adjustment unit 42E of the upper-stage medical assistance device 4E. That is, the two length adjustment units 42E and 42F rotate in conjunction with each other.

According to the present embodiment, the two length adjustment units 42E and 42F can adjust the use lengths of the medical devices 51 of different types from each other. For example, in the medical assistance device 4D illustrated in Fig. 10, the upper-stage medical assistance device 4E can adjust the use length of the catheter. In addition, the lower-stage medical assistance device 4F can adjust the use length of the guide wire. Accordingly, the choice of the procedure in the biological lumen can be widened. Moreover, even in a case where electricity is not provided, the surgeon 32 or the like rotates the length adjustment unit 42E using the concave portion 421E of the upper-stage length adjustment unit 42E, and thus, can rotate the lower-stage length adjustment unit 42F and can adjust the use lengths of the medical devices 51 of different types from each other.

Note that when the surgeon 32 or the like separates the upper-stage medical assistance device 4E and the lower-stage medical assistance device 4F from each other, the upper-stage length adjustment unit 42E and the lower-stage length adjustment unit 42F can rotate independently of each other. In addition, the number of concave portions 421E is not limited to 4 and the number of concave portions 421F is not limited to 4. That is, the number of concave portions 421E may be three or less or may be five or more and the number of concave portions 421F may be three or less or may be five or more.

Moreover, in the medical assistance device 4D illustrated in Fig. 10, the two length adjustment units 42E and 42F are provided rotatably with respect to the supports 41E and 41F about the support shaft 411 extending in the vertical direction. However, installation forms of the two length adjustment units 42E and 42F are not limited to this. For example, the two length adjustment units 42E and 42F may be provided rotatably with respect to the supports 41E and 41F about the support shaft 411 extending in the horizontal direction. In this case, it is preferable that the two length adjustment units 42E and 42F are disposed to be arranged in the horizontal direction, not in the up-down direction. That is, in this case, it is preferable that the two medical assistance devices 4E and 4F are stacked in the horizontal direction, not in the up-down direction and are coupled to each other.

According to this, the surgeon 32 or the like easily inserts fingers into both the concave portions 421E of the length adjustment unit 42E and the concave portions 421F of the length adjustment unit 42F and can easily rotate both the length adjustment unit 42E and the length adjustment unit 42F. Moreover, in this case, each of the two length adjustment units 42E and 42F can have the grip portion 421 (refer to Fig. 3) protruding outward from a surface thereof. Accordingly, the surgeon 32 or the like can easily hold the grip portion 421 which is provided in each of the two length adjustment units 42E and 42F and can easily rotate the two length adjustment units 42E and 42F.

Hereinbefore, the embodiments of the present invention are described. However, the present invention is not limited to the embodiments, and various changes can be made without departing from the scope of the claims. The configurations of the embodiments can be partially omitted and can be voluntarily combined so as to be different from the above.

### Reference Signs List

- 2: catheter room
- 4, 4A, 4B, 4C, 4D, 4E, 4F: medical assistance device
- 21: blood vessel imaging device
- 22: catheter table
- 23: digital imaging device
- 24: electrocardiogram
- 31: patient
- 32: surgeon
- 33: assistant
- 41, 41A, 41C, 41E, 41F: support
- 42, 42E, 42F: length adjustment unit
- 43: connection section
- 44: biasing unit
- 45: lock mechanism
- 46: coupling portion
- 47: elongated body
- 51: medical device
- 52: medical device holder
- 53: another medical device
- 411: support shaft
- 412: opening portion
- 413: introduction portion
- 414: derivation portion
- 421: grip portion
- 421E, 421F: concave portion
- 451: operation unit
- 452: roller
- 453: pressing portion
- 454: first shaft
- 455: second shaft
- 461: first rotation drive unit
- 462: rotation detection unit
- 463: control unit
- 464: second rotation drive unit

## Claims

1. A medical assistance device (4, 4A, 4B, 4C, 4D, 4E, 4F) which is connected to a medical device (51) which is inserted into a biological lumen and used in a procedure in the biological lumen, the medical assistance device (4, 4A, 4B, 4C, 4D, 4E, 4F) comprising:
a support (41, 41A, 41C, 41E, 41F);
a length adjustment unit (42, 42E, 42F) which is rotatably provided about a support shaft (411) with respect to the support (41, 41A, 41C, 41E, 41F) and performs at least one of winding and unwinding of the medical device (51) to adjust a use length of the medical device (51);
an elongated body which is attached to the length adjustment unit (42, 42E, 42F) and is connected to the medical device (51), and of which at least one of winding and unwinding is performed with respect to the length adjustment unit; and
a connection section (43) which is provided on an end portion of the elongated body and connects the end portion of the elongated body and an end portion of the medical device (51) to each other;
a rotation drive unit which is provided in the support (41, 41A, 41C, 41E, 41F) and generates a rotation force for rotating the length adjustment unit (42, 42E, 42F) when a voltage is supplied to the rotation drive unit;
**characterized in that** the medical assistance device further comprises:
a rotation detection unit (462) which detects a rotation angle of the length adjustment unit (42, 42E, 42F); and
a control unit (463) which receives a signal transmitted from the rotation detection unit (462) and calculates a winding length and an unwinding length of the elongated body (47) from a predetermined position, based on the rotation angle detected by the rotation detection unit (462).

2. The medical assistance device (4, 4A, 4B, 4C, 4D, 4E, 4F) according to claim 1,
wherein based on the rotation angle detected by the rotation detection unit (462), the control unit (463) controls the rotation drive unit to unwind the elongated body (47) when the control unit (463) detects a force in a direction in which the elongated body (47) is unwound, and the control unit (463) controls the rotation drive unit to wind the elongated body (47) when the control unit (463) detects a force in a direction in which the elongated body (47) is wound.

3. The medical assistance device (4, 4A, 4B, 4C, 4D, 4E, 4F) according to any one of claims 1 to 2,
wherein the rotation force for rotating the length adjustment unit (42, 42E, 42F) is a first rotation force,
wherein the rotation drive unit for generating the first rotation force is a first rotation drive unit (461), and
wherein a second rotation drive unit which generates a second rotation force for rotating the elongated body (47) about an axis of the elongated body (47) is further provided.

4. The medical assistance device (4, 4A, 4B, 4C, 4D, 4E, 4F) according to any one of claims 1 to 3,
wherein at least one of the support (41, 41A, 41C, 41E, 41F) and the length adjustment unit (42, 42E, 42F) has an introduction portion (413) which is spatially connected to the connection section (43) and into which another medical device (53) different from the medical device (51) is inserted or a working fluid used at the time of the procedure is fed.

## Patentansprüche

1. Medizinische Unterstützungsvorrichtung (4, 4A, 4B, 4C, 4D, 4E, 4F), die mit einer medizinischen Vorrichtung (51) verbunden ist, welche in ein biologisches Lumen eingeführt und bei einem Verfahren in dem biologischen Lumen verwendet wird, wobei die medizinische Unterstützungsvorrichtung (4, 4A, 4B, 4C, 4D, 4E, 4F) umfasst:
einen Träger (41, 41A, 41C, 41E, 41F);
eine Längeneinstelleinheit (42, 42E, 42F), die in Bezug auf den Träger (41, 41A, 41C, 41E, 41F) drehbar um eine Trägerwelle (411) vorgesehen ist und mindestens eines von Aufwickeln und Abwickeln der medizinischen Vorrichtung (51) durchführt, um eine Gebrauchslänge der medizinischen Vorrichtung (51) einzustellen;
einen langgestreckten Körper, der an der Längeneinstelleinheit (42, 42E, 42F) angebracht und mit der medizinischen Vorrichtung (51) verbunden ist, und von dem mindestens eines von Aufwickeln und Abwickeln in Bezug auf die Längeneinstelleinheit durchgeführt wird; und
einen Verbindungsabschnitt (43), der an einem Endabschnitt des langgestreckten Körpers vorgesehen ist und den Endabschnitt des langgestreckten Körpers und einen Endabschnitt der medizinischen Vorrichtung (51) miteinander verbindet;
eine Drehantriebseinheit, die in dem Träger (41, 41A, 41C, 41E, 41F) vorgesehen ist und eine Drehkraft zum Drehen der Längeneinstelleinheit (42, 42E, 42F) erzeugt, wenn eine Spannung an die Drehantriebseinheit angelegt wird;
**dadurch gekennzeichnet, dass** die medizinische Unterstützungsvorrichtung ferner umfasst:
eine Dreherfassungseinheit (462), die einen Drehwinkel der Längeneinstelleinheit (42, 42E, 42F) erfasst; und
eine Steuerungseinheit (463), die ein Signal empfängt, das von der Dreherfassungseinheit (462) übertragen wird, und auf der Grundlage des durch die Dreherfassungseinheit (462) erfassten Drehwinkels eine Aufwickellänge und eine Abwickellänge des langgestreckten Körpers (47) von einer vorbestimmten Position aus errechnet.

2. Medizinische Unterstützungsvorrichtung (4, 4A, 4B, 4C, 4D, 4E, 4F) nach Anspruch 1,
wobei die Steuerungseinheit (463) auf der Grundlage von dem von der Dreherfassungseinheit (462) erfassten Drehwinkel die Drehantriebseinheit steuert, um den langgestreckten Körper (47) abzuwickeln, wenn die Steuerungseinheit (463) eine Kraft in einer Richtung erfasst, in welche der langgestreckte Körper (47) abgewickelt wird, und die Steuerungseinheit (463) die Drehantriebseinheit steuert, um den langgestreckten Körper (47) aufzuwickeln, wenn die Steuerungseinheit (463) eine Kraft in einer Richtung erfasst, in welche der langgestreckte Körper (47) aufgewickelt wird.

3. Medizinische Unterstützungsvorrichtung (4, 4A, 4B, 4C, 4D, 4E, 4F) nach einem der Ansprüche 1 bis 2,
wobei die Drehkraft zum Drehen der Längeneinstelleinheit (42, 42E, 42F) eine erste Drehkraft ist,
wobei die Drehantriebseinheit zur Erzeugung der ersten Drehkraft eine erste Drehantriebseinheit (461) ist, und
wobei ferner eine zweite Drehantriebseinheit vorgesehen ist, welche eine zweite Drehkraft zum Drehen des langgestreckten Körpers (47) um eine Achse des langgestreckten Körpers (47) erzeugt.

4. Medizinische Unterstützungsvorrichtung (4, 4A, 4B, 4C, 4D, 4E, 4F) nach einem der Ansprüche 1 bis 3,
wobei mindestens eines von dem Träger (41, 41A, 41C, 41E, 41F) und der Längeneinstelleinheit (42, 42E, 42F) einen Einführungsabschnitt (413) aufweist, der mit dem Verbindungsabschnitt (43) räumlich verbunden ist, und in den eine weitere medizinische Vorrichtung (53), die sich von der medizinischen Vorrichtung (51) unterscheidet, eingeführt wird oder in den ein zu dem Zeitpunkt des Verfahrens verwendetes Arbeitsfluid eingespeist wird.

## Revendications

1. Dispositif d'assistance médicale (4, 4A, 4B, 4C, 4D, 4E, 4F) qui est connecté à un dispositif médical (51) qui est inséré dans une lumière biologique et utilisé dans une procédure dans la lumière biologique, le dispositif d'assistance médicale (4, 4A, 4B, 4C, 4D, 4E, 4F) comprenant :
un support (41, 41A, 41C, 41E, 41F) ;
une unité d'ajustement de longueur (42, 42E, 42F) qui est disposée de manière rotative autour d'un arbre de support (411) par rapport au support (41, 41A, 41C, 41E, 41F) et effectue au moins l'un d'un enroulement et d'un déroulement du dispositif médical (51) pour ajuster une longueur d'utilisation du dispositif médical (51) ;
un corps allongé qui est fixé à l'unité d'ajustement de longueur (42, 42E, 42F) et est connecté au dispositif médical (51), et dont au moins l'un d'un enroulement et
d'un déroulement est effectué par rapport à l'unité d'ajustement de longueur ; et
une section de connexion (43) qui est disposée sur une partie d'extrémité du corps allongé et connecte la partie d'extrémité du corps allongé et une partie d'extrémité du dispositif médical (51) l'une à l'autre ;
une unité d'entraînement en rotation qui est disposée dans le support (41, 41A, 41C, 41E, 41F) et génère une force de rotation pour faire tourner l'unité d'ajustement de longueur (42, 42E, 42F) lorsqu'une tension est appliquée à l'unité d'entraînement en rotation ;
**caractérisé en ce que** le dispositif d'assistance médicale comprend en outre :
une unité de détection de rotation (462) qui détecte un angle de rotation de l'unité d'ajustement de longueur (42, 42E, 42F) ; et
une unité de commande (463) qui reçoit un signal transmis par l'unité de détection de rotation (462) et calcule une longueur d'enroulement et une longueur de déroulement du corps allongé (47) à partir d'une position prédéfinie, sur la base de l'angle de rotation détecté par l'unité de détection de rotation (462).

2. Dispositif d'assistance médicale (4, 4A, 4B, 4C, 4D, 4E, 4F) selon la revendication 1,
dans lequel, sur la base de l'angle de rotation détecté par l'unité de détection de rotation (462), l'unité de commande (463) commande l'unité d'entraînement en rotation pour dérouler le corps allongé (47) lorsque l'unité de commande (463) détecte une force dans une direction dans laquelle le corps allongé (47) est déroulé, et l'unité de commande (463) commande l'unité d'entraînement en rotation pour enrouler le corps allongé (47) lorsque l'unité de commande (463) détecte une force dans une direction dans laquelle le corps allongé (47) est enroulé.

3. Dispositif d'assistance médicale (4, 4A, 4B, 4C, 4D, 4E, 4F) selon l'une quelconque des revendications 1 à 2, dans lequel la force de rotation pour faire tourner l'unité d'ajustement de longueur (42, 42E, 42F) est une première force de rotation,
dans lequel l'unité d'entraînement en rotation pour générer la première force de rotation est une première unité d'entraînement en rotation (461), et
dans lequel une seconde unité d'entraînement en rotation qui génère une seconde force de rotation pour faire tourner le corps allongé (47) autour d'un axe du corps allongé (47) est en outre prévue.

4. Dispositif d'assistance médicale (4, 4A, 4B, 4C, 4D, 4E, 4F) selon l'une quelconque des revendications 1 à 3, dans lequel au moins l'un parmi le support (41, 41A, 41C, 41E, 41F) et l'unité d'ajustement de longueur (42, 42E, 42F) comporte une partie d'introduction (413) qui est connectée spatialement à la section de connexion (43) et dans laquelle un autre dispositif médical (53) différent du dispositif médical (51) est inséré ou dans laquelle un fluide de travail utilisé au moment de la procédure est acheminé.
